# EUROPEAN PATENT APPLICATION

(11) **EP 2 198 823 A1**
(43) Date of publication of application: **23.06.2010**
(21) Application number: 08172441.1
(22) Date of filing: 19.12.2008
(51) Int. Cl.: A61H 31/00, A61M 16/00

(54) **Ventilation device with CPR mode**

(71) Applicant: Dräger Medical B.V., 5683 CR Best (NL)
(72) Inventor: de Jong, Franciscus, 5685 GE, Best (NL)
(74) Representative: DeltaPatents B.V.

(57) **Abstract**

A ventilation device, for forcing a respiratory gas into a patient, has a flow sensor for sensing a flow of the respiratory gas at least into or out of the patient. A controller counts a number of cycles of the flow, and generates a control signal under control of a specific predetermined CPR procedure and of the number of cycles counted. The ventilation device has a signaling interface for receiving the control signal and for, in response thereto, generating an output indicative of a phase in the pre-determined CPR procedure.

## Description

### FIELD OF THE INVENTION

The invention relates to a medical ventilation device for forcing air into a lung of a patient.

### BACKGROUND ART

Cardiopulmonary Resuscitation (CPR) is a well known medical procedure, performed by emergency response personnel or laypersons trained in first-aid procedures, to save the life of a victim of cardiac arrest or respiratory arrest. The aim of CPR is to sustain the blood circulation and respiration in an artificial way via chest compressions and lung ventilation until the victim's heart starts beating again. According to established medical practice, ventilation (or: inflation) of the lungs using a respiratory gas should occur during that phase of the compression cycle, wherein the heart is allowed to expand (i.e., during the diastole period).

Known ventilation devices (also referred to in this context as ventilator, or resuscitator) do not function well while chest compressions are being applied. The known device follows a pre-determined, fixed ventilation procedure, and the person, e.g., a paramedic, who is applying the chests compressions to the patient, has to synchronize with this procedure. Applying chest compressions is a task that wears out any person very fast and the synchronizing quickly becomes harder and harder. Compressions of the chest may lead to high pressure peaks in the patient's airways that usually give rise to alarms in the ventilation device. If the person continues to apply the chest compressions asynchronously with the ventilation cycles of the ventilation device, there is a risk that the pressure in the lungs becomes extremely high, leading to irreversible lung damage.

Synchronization between chest compressions and ventilation can solve the problem of high lung-pressures. Apparatus for automatic control of both chest compression and ventilation are disclosed in, e.g., EP 0 029 352, EP 1 820 484 and in US 4,397,306.

EP 0 029 352 discusses various CPR procedures for employing ventilation, and advocates a specific one thereof. This publication discloses an apparatus for conducting the specific CPR protocol. The apparatus comprises a combination of a reciprocal cardiac compressor means for cyclically compressing a patient's chest and a ventilating means for inflating the patient's lungs to a relatively benign limiting pressure over a period of time encompassing at least one cycle of the compressor means. Both the cardiac compressor and the ventilator of the CPR apparatus are pneumatically operated and pneumatically controlled. In order to set the apparatus up, the only power source required is an external source of compressed gas, normally oxygen. This supply of pressurized oxygen operates the apparatus. Ventilator output control means is provided, which both prevents retrograde and exhale flow during the systolic portion of the cycle of the compression means, thus providing for a pressure increase in the patient's lungs due to external cardiac massage, and periodically vents the patient's lungs to the atmosphere.

A ventilator subassembly is integrally mounted with the compressor. A breathing hose is connected to an air outlet and has a mask or tube for directing oxygen enriched air into the patient's lungs. Compressor cycles are controlled by a valve, and periodic output pulses of oxygen from the valve are allowed to pass to the ventilator. These pulses activate a programmer valve within the ventilator to turn it on periodically and the duration of the "on"-cycle is regulated by a timer control. Oxygen also passes to a pneumo-mechanical device serving to periodically open a passageway for a flow of oxygen to the ventilator at regular intervals. This device can be preset to provide flow of oxygen to the ventilator at regular multiples of compressor cycles (as done in most known CPR techniques), or may be preset to provide a pulse of oxygen at a preset limiting pressure that extends over a plurality of compressor cycles.

The output of the ventilator is controlled by a ventilator output control means, which prevents retrograde flow back to the ventilator and exhale flow from the patient's lungs during systolic portions of the compressor cycle. Blocking retrograde and exhale flow during systole provides a pressure increase in the patient's lungs due to compression of the patient's chest. The ventilator output control means further serves to periodically vent the patient's lungs to the atmosphere.

EP 1 820 484 discloses an apparatus for automatic delivery of chest compressions and ventilation to a patient. The apparatus has a chest compressing device configured to deliver compression phases during which pressure is applied to compress the chest, and decompression phases during which approximately zero pressure is applied to the chest. The apparatus further has a ventilator configured to deliver positive, negative, or approximately zero pressure to the airway. The apparatus also includes control circuitry and a processor, wherein the circuitry and processor are configured to cause the chest compressing device to repeatedly deliver a set containing a plurality of systolic flow cycles. Each systolic flow cycle comprises a systolic decompression phase and a systolic compression phase, and at least one diastolic flow cycle interspersed between sets of systolic flow cycles. Each diastolic flow cycle comprises a diastolic decompression phase and a diastolic compression phase, wherein the diastolic decompression phase is substantially longer than the systolic decompression phase.

US 4,397,306 describes synchronization of the ventilation cycle with the compression cycle. An automatic chest compression device is synchronized with an automatic ventilator. High pressure ventilation pulses are delivered simultaneously with the compression phase (i.e., when chest pressure is applied). Slightly negative ventilation pulses are delivered simultaneously with the decompression phase (i.e., when no chest pressure is applied). The negative ventilation pulses are assumed to move greater amounts of blood into the chest during diastole. Also, a conventional ventilation cycle is introduced every approximately sixth compression / decompression cycle, when no compression is occurring. This is said to be valuable for sufficient alveolar gas exchange since very little air flow occurs during the positive ventilation pressure cycles that are synchronized to the compression phase.

For completeness, US 4,676,232 discloses a method and device for providing respiration to a patient. The respiration is synchronized with the heart rhythm, so that the pressure exerted on the heart can be adjustably increased to such a degree that the contraction of the heart can be significantly increased without injury occurring to the lungs and/or to the thorax walls. The desired pressure increase around the heart by means of synchronizing the ventilation with the heart rhythm is assumed to be at least partially cancelled thereby, as a result of which the chest and the abdomen are allowed to expand during the step of respiration. Therefore, the expansion is restricted in that a uniform force is exerted on the chest and/or on the abdomen. Upon respiration, the lungs can be partially expanded toward the inside so that the pressure increase around the heart is achieved to the desired degree. The respiration of the patient should thereby occur at a particular point of time in the heart rhythm. That means that one breath should be present per heart cycle or on the other hand, that the breath respectively appears only on every second heartbeat or even more infrequently but synchronous with the heart activity in each case. This publication also discloses a respirator having a first valve control arrangement for controlling the flow of the respiratory gas from a source to and from the patient, means for generating and exerting a uniform pressure on the chest and/or the abdomen, means for sensing the parameters of the heart activity and control means which actuate at least the valves of the first arrangement as a function of the sensed signal.

### SUMMARY OF THE INVENTION

The inventor has realized that a major disadvantage of the known systems is that they require that both a compression unit and a ventilation device be present, and controlled via a central control unit. In practice, however, such a complete system is often not available at the scene where it is needed outside a hospital. Moreover, it is not practical for emergency personnel to carry too much equipment with them as this hampers their speed, mobility and agility. For example, a rapid response paramedic in an urban area is often dispatched to the scene of a traffic accident on a motorcycle. Such a motorized paramedic is better capable of moving over heavily congested roads than a fully equipped ambulance van. However, a motorcycle is limited with regard to volume and weight of what can be stowed onboard. Similarly, an ambulance helicopter needs to be agile and relatively light-weight as well. As a consequence, there is a need to synchronize the actions of chest compression and ventilation under less than ideal circumstances, without extra equipment beyond the bare minimum.

The known ventilation devices, which are currently commercially available, do not have a special operating mode dedicated to CPR procedures. The advised CPR setting of the ventilation device, which can be chosen via the ventilation device's user-control interface, is a fixed breathing mode, of e.g., 12 breaths per minute, at a very high maximum pressure limiting value. This passive setting allows a very high air volume per minute and at the same time prevents the ventilation device to give alarms as a result of high pressure peaks. Some known ventilation devices have this setting indicated as "CPR mode" among the operational mode options that can be selected, e.g., by means of a rotatable knob in the device's user-control interface.

The inventor now proposes a ventilation device with a new ventilation mode that plays an active role in the CPR procedure. In an embodiment, the ventilation device makes use of the measurement of the airflow that is going through the patient airways. When the chest is being compressed, air will flow out of the lungs. This can be detected by an air flow sensor and/or a pressure sensor, and can be used to determine, or further control, the ventilation actions in the new ventilation mode. A CPR protocol determines the compression rate, the number of compressions and the number and length of the ventilation cycles. In the new ventilation mode, the relevant CPR protocol is chosen from among a plurality of such protocols available for programming the operation of the ventilation device. The ventilation device, thus programmed, signals to the rescuer when to carry out the compressions of the chest. The display of the ventilation device gives information to the rescuer about the current phase in the CPR protocol. On basis of the CPR protocol chosen, the ventilation device determines when and how the patient is to be ventilated between the compression cycles. The ventilation device can signal this to the human operator. The invention thus contributes to optimizing treatment of the blood flow circulation and breathing assistance. In addition to signaling compression information to the rescuer, this approach can also be used to synchronize with further equipment configured for performing chest compressions. The synchronization is based on the following. The ventilation device of the invention detects the temporal pattern of the chest compressions performed by an automated chest compression apparatus. The ventilation device is configured for using this detected pattern in order to synchronize with the chest compression apparatus, without the need for dedicated signaling or data communication between the ventilation device and the chest compression apparatus. The synchronizing is performed by the ventilation device of the invention under control of software onboard the device. Alternatively, a dedicated signaling or data communication interface is provided at the ventilation device of the invention for communicating with the chest compression apparatus so as to be able to synchronize.

More specifically, the invention relates to a medical ventilation device with a CPR mode configured for forcing a respiratory gas into a patient. The medical ventilation device comprises a sensor input for connection to a sensor that is operative to sense a physical quantity representative of occurrences of chest compressions applied to the patient. The sensor provides a sensor output signal for receipt at the sensor input of the ventilation device. The ventilation device also comprises a controller functionally coupled to the other sensor input for counting a number of the occurrences, and for generating a control signal under control of a specific pre-determined procedure and of the number counted. The ventilation device further has a signaling interface for receiving the control signal and for in response thereto generating an output indicative of a phase in the pre-determined procedure.

In an embodiment of the ventilation device, the sensor input comprises at least one of the following: a flow sensor input for connection to a flow sensor that is operative to sense a flow of the respiratory gas at least into or out of the patient; a pressure sensor input for connection to a pressure sensor that is operative to sense a pressure of the flow of the respiratory gas; a further sensor input for connection to a further sensor that is operative to sense a pressure or force applied to the patient; and another sensor input for connection to an accelerometer that is operative to sense accelerations of an agent applying the chest compression.

Thus, recurrent changes in the quantity sensed (volume, gas pressure, mechanical pressure, force, acceleration, etc.) are indicative of the chest compression cycles. Keeping track of these recurrent changes enables the controller to determine the phase of the CPR procedure carried out on the patient. The signaling interface provides information about the current phase of the pre-determined procedure on the basis of the number of compression cycles counted and derived from the measurements via the sensor. This information can be made available to the rescuer via the signaling interface at the ventilation device. Based on this information, the rescuer can control the manual chest compression process.

According to current insights, it is more important to initiate or assist the circulation of the patient's blood than to initiate or assist lung ventilation. In the absence of lung ventilation, a typical adult person can survive for about 6 minutes without damage if the blood circulation is kept going, thus relying solely on the oxygen carried by the person's blood. A rescuer will therefore first pay attention to starting or assisting of the blood circulation. By means of the measurements, e.g., via the gas flow sensor and/or the pressure sensor, the ventilation device of the invention can determine which CPR protocol the rescuer is following and adjust accordingly, eventually signaling to the rescuer when to apply lung ventilation.

In addition, the ventilator's signaling interface can also be configured for communicating with other apparatus, e.g., a chest compression unit, via a wired or wireless connection.

An embodiment of the ventilation device in the invention has a configuration interface for configuring the controller through selection of the specific pre-determined procedure from a plurality of pre-determined procedures. This enables the rescuer to select the most suitable one of the pre-determined procedures, e.g., 30:2 or 15:2 CPR procedures, in view of the actual circumstances in operational use of the ventilation device.

In a further embodiment, the signaling interface comprises at least one of a graphical user interface, an auditory user interface, a tactile user interface. The information about the current phase in the procedure can thus be communicated to the rescuer in a visual, audible or tactile manner.

In a further embodiment, the ventilation device is configured for synchronization with a chest compression apparatus under control of the sensor output signal. For example, if the ventilation device of the invention has a pressure sensor input for connecting to a pressure sensor that is operative to sense the pressure of the respiratory gas flow, the ventilation device can also be configured for automatic synchronization with the chest compression apparatus through detection of changes in the pressure. Accordingly, the ventilation device of the invention can be used as a modular building block of a CPR system for combined control of chest compressions and ventilation cycles.

In an embodiment of the invention, the ventilation device is configured for synchronization with a specific one of a plurality of chest compression apparatus. The configuration interface enables to select the specific chest compression apparatus, e.g., type and or brand, so as to render the operation of the ventilation device compatible with the selected chest compression apparatus. In an embodiment of such a ventilation device the selection is to be done manually by the human operator, e.g., through a suitable menu of options. Each respective one of the options represents a respective type or brand of a chest compression apparatus. In another embodiment, the ventilation device is configured for data communication with a chest compression apparatus that itself is also configured for data communication. The ventilation device of the invention requests the chest compression apparatus to communicate information about its type or brand, or its functional control interface. This information then enables the ventilation device to automatically configure itself for synchronization with the chest compression apparatus.

In a further embodiment of the invention, the controller is operative to advance (or delay) the generating of the output in the signaling interface with respect to the phase in the pre-determined procedure so as to help a human operator synchronizing with the procedure. The controller compares the occurrences of the chest compressions as sensed with the occurrences of the compressions as specified by the pre-determined procedure. If the human operator begins to lag behind and does not keep up with the recommended pace of the chest compressions according to the procedure, the controller advances the output from the signaling interface in dependence on the delay measured so as to drag the human operator towards the specified rhythm of chest compressions.

As mentioned above, some embodiments of the ventilation device in the invention rely, in operational use, on the presence of at least one of a flow sensor and a pressure sensor for determining the occurrences of chest compressions based on the pattern of changes in the flow of the respiratory gas and/or in the pressure of the respiratory gas as sensed. As a flow sensor and/or pressure sensor is typically a standard component of a medical ventilation device, above embodiments represent an elegant solution in that the features already present are now being used for the purpose of implementing the CPR mode. However, alternative ways are feasible of implementing the CPR mode with a medical ventilation device of the invention, wherein a physical quantity is being monitored different from an attribute of the respiratory gas. What is needed then is that the ventilation device has an input for another sensor that senses changes in the magnitude of a physical quantity, the changes being representative of the occurrences of the chest compressions. This then also enables the ventilation device to keep track of the phase in the CPR protocol. For example, a sensor can be used that senses the changes in the force or pressure applied by the rescuer to the chest of the patient. The magnitude of the force or pressure as sensed has a cyclic pattern from which the occurrences of the chest compressions can be inferred. Such a force sensor or pressure sensor could be located in or at , e.g., a pillow or blanket put underneath the patient, in a pad put on top of the chest of the patient and forming a mechanical interface between the rescuer and the patient. Such a sensor could also be integrated with agent applying the chest compressions, e.g., with the gloves that are worn by the rescuer or mounted on the mechanical interface of the chest compression apparatus that applies the pressure to the patient's chest. Yet another sensor is based on registering accelerations of the hands of the rescuer or of the mechanical interface of the chest compression apparatus. The cyclic or repetitive pattern of accelerations can again be used to infer the occurrences of the chest compressions and therefore the occurrences of the changes in the respiratory flow. The sensor communicates wirelessly or in a wired fashion with the ventilation device configured for receipt and proper processing of the signals from the sensor. An embodiment of the ventilation device in the invention is therefore equipped with a proper interface for receipt of the pressure or force related signals from the pressure or force sensor.

### BRIEF DESCRIPTION OF THE DRAWING

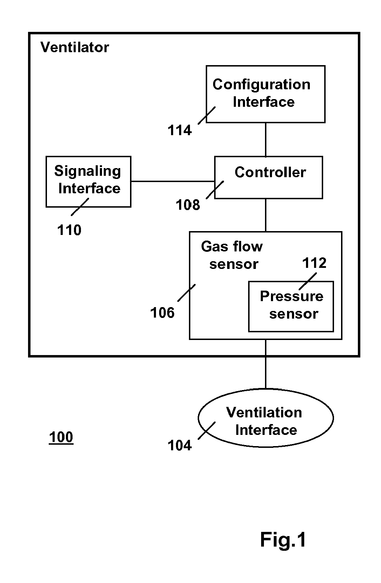

The invention is explained in further detail, by way of example and with reference to the accompanying drawing, wherein Fig.1 is a block diagram of a medical ventilation device of the invention.

### DETAILED EMBODIMENTS

In the invention, operation of a ventilation device for breathing assistance to a patient is controlled via the pressure or the volume of the flow of the respiratory gas. The ventilation device has a pressure sensor and a flow sensor. With a properly placed flow sensor (usually close to the mouth of the patient), it is possible to measure the inspiratory flow and/or the expiratory flow. In case of CPR, an operational mode of the ventilation device is created, wherein the PEEP (Positive End Expiration Pressure) is 0 mbar or higher. In the absence of a spontaneous breathing action from the patient, there is no flow between the patient and the ventilation device. A compression of the chest results in an expiration flow that can easily be detected. This allows the ventilation device to count the number of compressions as well as determine the frequency, or another temporal attribute, of the sequence of compressions.

The ventilation device in the invention has a user control interface, e.g., a graphical user interface (GUI), that can show the progress made according to the CPR procedure. The following example scenario illustrates the advantage of such a system. The rescuer selects a CPR mode at the ventilation device. The ventilation device has a fixed CPR procedure, or multiple CPR procedures from which the rescuer can select the one best suited to the situation. Selection is made, e.g., via the GUI.

The selected procedure is, e.g., the well-known 30:2 protocol, recommended for single rescuers. Herein, the 30:2 indication refers to the compression-ventilation ratio of 2 ventilation cycles per 30 compression cycles. The GUI then displays the number 30 at the start and, after each compression step measured, the displayed number is decremented by unity. The last three steps are accompanied by an audible sound and/or a visible warning and/or a tactile warning. When the counter arrives at the number zero, the person performing the chest compressions knows he/she has to stop. The ventilation device subsequently performs 2 ventilation cycles of, e.g., 1 second, after which a new sequence of 30 chest-compression cycles can be started by the rescuer. The ventilation device can furthermore give an audible, visible or tactile signal in case the rescuer is carrying out the compressions at a frequency that deviates from the frequency that is advisable, given the circumstances. Thus, by providing visual and/or auditory and or tactile feedback, the ventilation device used in CPR assists the human operator to adhere to the recommended CPR protocol.

In addition, the ventilation device can use the short time period between two successive compressions to give inspiration pulses. As known, these inspiration pulses improve the gas exchange inside the lungs. If these inspiration pulses are slightly delayed, e.g., by 50 -100 msec, a negative pressure will occur for a short time, which facilitates diffusion of the gas in the lungs.

Measuring the respiratory gas flow also enables to calculate the total respiratory minute volume (MV). The MV is a physiological quantity representative of the volume of gas that is inhaled or exhaled from a person's lungs in one minute. The MV as calculated can be displayed at the GUI so that the rescuer can decide if sufficient air exchange is taking place.

The GUI can also show the frequency of the chest compressions. If necessary, an audible signal can be used to assist the rescuer with applying the compressions in the recommended temporal pattern of about 100 compressions per minute. A visual, auditory or tactile signal can be used to signal deviation from the ideal frequency.

The compression force cannot be measured directly via measuring the gas flow. However, the peak-flow and the expiratory volume per compression step can be used to give an indication of the magnitude of the force or pressure applied in compressing the victim's chest. This information can also be shown on the GUI so that the rescuer can determine if the force applied is more or less of the desired magnitude, or whether a stronger or weaker force is necessary to compress the victim's chest.

Above scenarios are centered on the ventilation device giving guidance to a human operator performing the chest compression. The ventilation device of the invention can similarly be used to cooperate with a mechanical compression unit.

An attractive feature of the ventilation device of the invention resides in the fact that a communication link between the ventilation device and compression unit is not necessary, because the ventilation unit can autonomously detect the status of the mechanical compression unit through the gas flow detection and respond accordingly. Preferably, the relevant properties of several compression units have been stored in advance in a look-up table in the ventilation device for ease of reference. Selecting a relevant one of the compression units via the user interface of the ventilation device configures the ventilation device to be compatible with the selected compression unit.

In addition to the user interface, discussed above, or instead of the human interface in other embodiments, the ventilation device has an interface for communicating with a mechanical chest compression unit, e.g., optically or electronically. For example, selecting a particular one of the compression units, whose properties have been stored at the ventilation device in advance, configures the ventilation device for communicating using a specific communication protocol involving, e.g., voltage, frequency, data rate, etc., compatible with the control properties of the compression unit selected.

The CPR mode can be used at the same time to switch automatically to 100% oxygen to improve the oxygenation of the blood. Typically, a ventilation device has a source of respiratory gas. This source supplies the respiratory gas at the required pressures and also determines the concentration of oxygen in the gas mixture. The ventilation device typically has a control for manually adjusting the percentage of oxygen in the respiratory gas supplied to the patient. An embodiment of the ventilation device of the invention has a CPR mode, wherein the ventilation device automatically and temporarily overrules the percentage as has been manually set, and supplies 100% oxygen.

Preferably, the ventilation device of the invention has a gas flow meter and this can help to resuscitate a patient as described above. That is, the flow meter measurements assist the rescuer with applying the chest compressions of correct frequency and magnitude. However, the ventilation device of the invention can also be exploited as a stand-alone gas flow meter. Such a gas flow meter could be a standard device, similar to a thermometer in a first aid kit. A small display and a tiny buzzer can help to follow the resuscitation protocol. One can imagine that such a flow meter is put in, or close to, the mouth of the patient. The design thereof is made such that a mouth-to-mouth breathing is possible. Preferably, the design is such that the victim's mouth is fully covered, thus limiting the risk of the rescuer contracting a contagious disease from the patient as a result of direct physical contact via the mouth.

Note that measuring the gas flow can support both the chest compressions and the ventilation. Note that there are a variety of flow meters that only measure flow but not pressure.

Fig.1 is a block diagram of a medical ventilation device 100 according to the invention. Ventilation device 100 is operative to force a respiratory gas into the lungs of a patient via a ventilation interface 104, e.g., a ventilation mask or an endotracheal tube. Ventilation device 100 has a gas flow sensor 106 that, in operational use of ventilation device 100, is connected to ventilation interface 104. Flow sensor 106 senses, for example, whether or not there is an airflow into (or out of, or both) the lungs. A controller 108, e.g., a microcontroller or other dedicated electronic circuitry, is functionally coupled to flow sensor 106 for receiving its output so as to count a number of cycles of the gas flow. If the chest compressions are repetitively applied in a certain temporal pattern, the gas flow assumes a similar repetitive pattern. Controller 108 is configured to generate a control output in dependence on the number of cycles counted and the CPR protocol selected. Controller 108 is furthermore configured to integrate over time the gas flow measurements from flow sensor 106 in order to determine the total respiratory minute volume (MV) and other derived quantities. Ventilation device 100 further includes a signaling interface 110 that receives the control output of controller 108. Signaling interface 110 generates a signal indicative of the number of cycles counted in response to the control output from the controller. Interface 110 includes, for example, a display monitor such as an LCD, for presenting visual feedback to the rescuer, and/or a loudspeaker for providing auditory feedback. Ventilation device 100 may comprise a pressure sensor 112 for sensing a pressure of the gas flow.

Controller 108 receives as input the output from flow sensor 106 and/or from pressure sensor 112, and the manual settings entered by the operator via configuration interface 114. On the basis of these inputs, controller 108 provides signals via the signaling interface 110, and may also control the percentage of oxygen supplied via ventilation interface 104, overruling the manual setting in the CPR mode as mentioned above. Controller 108 is implemented by means of dedicated hardware, a microcontroller or a data processor running dedicated software, or a combination thereof.

There are flow meters that measure both volume and pressure. In the embodiment shown, flow sensor 106 and pressure sensor 112 are illustrated as accommodated within the housing of ventilation device 100, combined with one another in a flow meter. In alternative embodiments (not shown), one or both of flow sensor 106 and pressure sensor 112 is/are positioned external to the housing of ventilation device 100, e.g., in ventilation interface 104, and connected to a proper input at the housing of ventilation device 100.

In a specific embodiment, interface 110 comprises tactile feedback means to provide to the rescuer information in a tactile modality. This may be advantageous in view of the fact that the rescuer has to watch the victim and perhaps to keep an eye on the environment because of potentially hazardous circumstances associated with an emergency situation, such as a fire, an approaching vehicle, risk of a building collapsing, etc. As the rescuer's auditory and visual senses are already occupied, it may therefore be more efficient to use tactile feedback for the signaling function. For example, the rescuer carries an actuator on his/her wrist that receives the signal wirelessly from the ventilation device and converts it into a patterned physical movement that helps the rescuer to synchronize with ventilation device 100. Such actuators are well known from, e.g., mobile telephones that provide vibro-tactile sensations such as the E770 handset from Samsung upon receiving a call or an SMS message. The force-feedback technology used has been developed by Immersion Corporation.

Preferably, ventilation device 100 comprises a configuration interface 114 through which the rescuer can select one of a plurality of CPR procedures for thereby configuring the generation of the signal provided through signaling interface 110. For example, configuration interface 114 comprises a touch screen through which a pre-determined menu of selectable options is accessible as represented in a GUI of interface 114. Interfaces 110 and 114 may, but need not, use the same GUI. The selectable options include, e.g., a variety of CPR protocols (e.g., the 30:2 protocol mentioned above, a 15:2 protocol, etc.), an input for configuring ventilation device 100 to be used for signaling to the human operator and an input for alternatively configuring device 100 for synchronizing with a mechanical compression unit (not shown) that is to take care of the chest compressions, inputs indicating the relevant type or brand of the mechanical compression unit being used for configuring ventilation device 100 for cooperation with a specific compression unit, etc. Further options may include an input to activate the inspiration pulses between two successive compression cycles; etc.

## Claims

1. A medical ventilation device (100) with a CPR mode configured for forcing a respiratory gas into a patient, and comprising:
a sensor input for connection to a sensor that is operative to sense a physical quantity representative of occurrences of chest compressions applied to the patient, and to provide a sensor output signal for receipt at the sensor input;
a controller (108) functionally coupled to the other sensor input for counting a number of the occurrences, and for generating a control signal under control of a specific pre-determined procedure and of the number counted; and
a signaling interface (110) for receiving the control signal and for in response thereto generating an output indicative of a phase in the pre-determined procedure.

2. The medical ventilation device of claim 1, wherein the sensor input comprises at least one of the following:
a flow sensor input for connection to a flow sensor (106) that is operative to sense a flow of the respiratory gas at least into or out of the patient;
a pressure sensor input for connection to a pressure sensor (112) that is operative to sense a pressure of the flow of the respiratory gas;
a further sensor input for connection to a further sensor that is operative to sense a pressure or force applied to the patient; and
another sensor input for connection to an accelerometer that is operative to sense accelerations of an agent applying the chest compression.

3. The ventilation device of claim 1 or 2, comprising a configuration interface (114) for configuring the controller through selection of the specific pre-determined procedure from a plurality of pre-determined procedures.

4. The ventilation device of claim 1, 2 or 3, wherein the signaling interface comprises at least one of a graphical user interface, an auditory user interface, a tactile user interface.

5. The ventilation device of claim 1, 2, 3 or 4, configured for synchronizing with a chest compression apparatus under control of the sensor output signal.

6. The ventilation device of claim 3, 4 or 5, wherein:
the ventilator device is configured for control of a specific one of a plurality of chest compression apparatus through the output of the signaling interface;
the configuration interface is configured for selecting the specific chest compression apparatus so as to render the ventilator device compatible with the selected chest compression apparatus.

7. The ventilation device of claim 1, 2 or 3, wherein:
the controller is operative to compare actual occurrences of the chest compression as sensed with advised occurrences as specified by the predetermined procedure; and
the controller is operative to advance or delay the generating of the output in the signaling interface with respect to the phase in the pre-determined procedure, in dependence on the comparison, for helping a human operator synchronizing with the pre-determined procedure.

8. The ventilation device of claim 2, comprising the flow sensor.

9. The ventilation device of claim 2, comprising the pressure sensor.

10. The ventilation device of claim 1, comprising an operator input for manually setting a percentage of oxygen in the respiratory gas to a specific value, and wherein the controller temporarily overrules the specific value set and supplies substantially 100% of oxygen as the respiratory gas.
